# EUROPÄISCHE PATENTSCHRIFT

(11) **EP 1 200 004 B1**
(45) Veröffentlichungstag und Bekanntmachung des Hinweises auf die Patenterteilung: **10.12.2003**
(21) Anmeldenummer: 00951323.5
(22) Anmeldetag: 30.06.2000
(51) Int. Cl.: A61B 19/02, A61L 2/26

(54) **Wiederverwendbarer und sterilisierbarer Sterilbehälter**
Reusable and sterilisable sterile container
Récipient stérile étant réutilisable et stérilisable

(30) Priorität: 30.07.1999 DE 19935986
(43) Veröffentlichungstag der Anmeldung: 02.05.2002
(73) Patentinhaber: Aesculap AG & Co. KG, 78532 Tuttlingen (DE)
(72) Erfinder: GABELE, Lorenz, 88605 Sauldorf (DE)
(74) Vertreter: Böhme, Ulrich, Dr. Dipl.-Phys.
(86) Internationale Anmeldenummer: EP0006124
(87) Internationale Veröffentlichungsnummer: WO01008583

(56) Entgegenhaltungen:
- EP-A- 0 513 614
- WO-A-92/00705
- WO-A-95/09579
- DE-A- 2 952 733
- US-A- 4 444 310
- US-A- 5 324 489
- US-A- 5 732 821

## Beschreibung

Die Erfindung betrifft einen wiederverwendbaren und wiedersterilisierbaren Sterilbehälter zur Aufnahme und sterilen Aufbewahrung insbesondere von chirurgischem Besteck oder Material mit einem durch einen Behälterboden und Behälterwände gebildeten Aufnahmeraum.

Medizinische Instrumente und insbesondere chirurgische Instrumente für den Stations- oder Ambulanzbedarf sind bis zu ihrem Gebrauch steril zu lagern. Dazu werden hauptsächlich Weichverpackungen verwandt, d.h. die Instrumente oder das Material ist in Sterilisierverpakkungen eingeschweißt. Das Eintüten von Instrumenten bzw. Material und das Verschweißen der Tüten kann zeitaufwendig sein.

Bisher bekannte Sterilbehälter weisen im Vergleich zu Weichverpakkungen einen relativ hohen Preis auf.

In der EP 0 513 614 A ist ein sterilisierfähiges Behältnis zur Aufnahme einzelner medizinischer oder chirurgischer Bestecke, Instrumente oder Geräte zum Zwecke des Sterilisieren und anschließenden sterilen Aufbewahrens beschrieben. Wenn dieser Behälter einmal geöffnet ist, sind alle darin enthaltenen Gegenstände unsteril, d.h. sie können nach einiger Zeit nicht mehr eingesetzt werden, ohne vorher erneut sterilisiert zu werden.

Weiterhin ist aus der WO 92/00705 A ein steriles Verpackungssystem bekannt, bei welchem eine Oberseite eines Blockes flach ist und mit einem Polyethylenfilm bedeckt ist. Dieser Film wird durch einen Deckel abgedeckt. Ein Gegenstand kann nur dann entnommen werden, wenn der Film mittels eines Werkzeuges zerrissen wird. Damit handelt es sich hier um ein Verpackungssystem, das nur einmal eingesetzt werden kann.

Es ist Aufgabe der Erfindung, einen gattungsgemäßen Sterilbehälter zu schaffen, weicher einfach und kostengünstig einsetzbar ist, nämlich mit dem sich eine Mehrzahl von chirurgischem Instrumentarium steril halten läßt und der Sterilbehälter dennoch einfach einsetzbar ist.

Diese Aufgabe wird bei einem gattungsgemäßen Sterilbehälter erfindungsgemäß dadurch gelöst, daß der Aufnahmeraum eine Mehrzahl getrennter Kammern umfaßt, daß jede Kammer einen eigenen Deckel als Verschlußelement aufweist und daß zwischen jeder Kammer und ihrem Verschlußelement eine Dichtung sitzt.

Durch die Ausbildung des Verschlußelements als Deckel läßt sich eine individuelle Kammer unabhängig von den anderen Kammern ohne Zerstörung des Verschlußelements öffnen und auch wieder schließen, und zwar mit demselben Verschlußelement. Durch das Vorsehen von einer Dichtung zwischen jeder Kammer und ihrem Verschlußelement läßt sich jede Kammer unabhängig von den anderen Kammern steril halten, so daß durch das Öffnen einer Kammer die Instrumente in den restlichen Kammern nicht unsteril werden. Die Kammern sind dann insofern abhängig voneinander, daß sie im selben Behälter angeordnet sind und somit sich gleichzeitig eine Vielzahl von Kammern sterilisieren lassen, während die Funktion der sterilen Kammern unabhängig voneinander ist.

Vor Gebrauch des steril aufbewahrten Bestecks bzw. Materials öffnet ein Benutzer nur das Verschlußelement jener Kammer, in dem das von ihm benötigte Instrument bzw. Material enthalten ist. Die anderen Kammern bleiben ungeöffnet und damit steril. Bei den bisher aus dem Stand der Technik bekannten Sterilbehältern war nachteilig, daß zur Entnahme eines Instrumentes der ganze Sterilbehälter geöffnet werden mußte und somit alle anderen Instrumente, sofern sie nicht sofort verwendet wurden, unsteril wurden.

Bei Operationen, bei denen eine definierte Art und/oder Anzahl von Instrumenten benötigt wird, beispielsweise bei Hals-Nasen-Ohren-Operationen, läßt sich ein entsprechend ausgebildeter erfindungsgemäßer Sterilbehälter mit dem benötigten Satz von Instrumenten bestücken und diese sind dann auf übersichtliche Weise für die Operation steril bereitgehalten.

Darüber hinaus läßt sich, im Gegensatz zu Tütenverpackungen, ein erfindungsgemäßer Sterilbehälter nach Reinigung und Desinfektion wiederverwenden.

Bei einer Variante einer Ausführungsform ist es vorgesehen, daß das Verschlußelement schwenkbar angeordnet ist. Dadurch läßt sich auf einfache Weise ein Schließen (zur sterilen Aufbewahrung eines Instrumentes oder von chirurgischem Material in der Kammer) bzw. Öffnen (zur Herausnahme) bewerkstelligen. Es kann auch vorgesehen sein, daß ein Verschlußelement mittels einer Schiebeführung angeordnet ist.

Ganz besonders vorteilhaft ist es, wenn das Verschlußelement zumindest teilweise durchsichtig ist. Auf diese Weise kann ein Bediener auf einfache Weise erkennen, was in einer Kammer gelagert ist. Es kann auch vorgesehen sein, daß der Sterilbehälter zumindest teilweise aus einem durchsichtigen Material gefertigt ist.

Ganz besonders vorteilhaft ist es, wenn die Dichtung unterdruckbeaufschlagbar ist. Dadurch läßt sich erreichen, daß sich das Verschlußelement oder ein Aufnahmeelement einer Kammer für die Dichtung (je nachdem welches Element die Dichtung hält) beim Schließen an der Dichtung festsaugt, um so eine besonders gute Abdichtung gegenüber dem Außenraum zu erreichen.

Es kann vorgesehen sein, daß der Sterilbehälter in dem Behälterboden und/oder äußeren Behälterwänden Öffnungen aufweist. Dadurch ist einerseits die Reinigung/Desinfektion erleichtert, da Reinigungs-/Desinfektionsfluide durch die Öffnungen abfließen können. Andererseits wird dadurch auch die Möglichkeit geschaffen, daß Kondenswasser oder Dampf aus einer geschlossenen Kammer austreten kann.

Es ist dann zur Abdeckung der Öffnungen ein Sterilfilter vorzusehen, bei dem es sich insbesondere um einen Dauersterilfilter handeln kann. Ein solches Sterilfilter verhindert das Eindringen von unsteriler Luft in die Kammern. Andererseits kann trotzdem noch aus den Kammern in den Außenraum Wasser oder Dampf über das Sterilfilter abgeführt werden, welches in einer Kammer angesammelt einen Nährboden für Keime darstellen kann.

Ganz besonders vorteilhaft ist es, wenn ein Sterilfilter und insbesondere ein einziges Sterilfilter für mehrere Kammern vorgesehen ist. Dadurch ist der Zeitaufwand und auch der konstruktive Aufwand zur Anordnung des Sterilfilters an dem Sterilbehälter vereinfacht und außerdem läßt sich das Sterilfilter schneller ersetzen als entsprechend mehrere solcher Filter.

Günstigerweise sitzt das Sterilfilter unterhalb des Behälterbodens. Dadurch läßt sich der Behälterboden mit den entsprechenden Öffnungen zur Dampf-/Wasserabfuhr bzw. zur Fluidabfuhr bei der Reinigung und Desinfektion versehen und auch bei eingelegten Instrumenten kann ein solcher Reinigungs- und Desinfektionsprozeß durchgeführt werden, wobei ein gutes Ablaufen der Fluide erreicht ist und die Instrumente nicht zusätzlich gehalten werden müssen.

Günstigerweise weist der erfindungsgemäße Sterilbehälter eine Halterung für ein Sterilfilter auf, um auf diese Weise Sterilfilter ersetzen zu können oder nach einem Reinigungsund Desinfektionsvorgang das Sterilfilter an dem erfindungsgemäßen Sterilbehälter anordnen zu können.

Es kann bei einer Variante einer Ausführungsform vorgesehen sein, daß die Halterung von dem Sterilbehälter abnehmbar ist. Dadurch kann unter Umständen der Reinigungs- und Desinfektionsprozeß erleichtert werden. Es kann aber auch vorgesehen sein, daß die Halterung schwenkbar angeordnet ist, um sie auf diese Weise bei einem Reinigungs- und Desinfektionsprozeß insbesondere vom Behälterboden wegzuschwenken.

Ganz besonders vorteilhaft ist es, wenn das Sterilfilter mittels eines Schnappverschlusses in der Halterung gehalten ist. Das Sterilfilter läßt sich dann auf einfache und schnelle Weise ersetzen und es wird andererseits sicher in der Halterung gehalten, um das Eindringen von unsteriler Luft in eine verschlossene Kammer zu verhindern.

Günstigerweise ist es vorgesehen, daß zwischen Sterilfilter und Behälter ein Schutzgitter sitzt. Dieses Schutzgitter hat die Aufgabe, eine Penetration des Sterilfilters durch insbesondere spitze Instrumente, welche in dem Sterilbehälter gelagert sind, zu vermeiden, d.h. das Sterilfilter vor Leckage zu schützen. Wenn das Schutzgitter dampfdurchlässig ist, dann kann Wasserdampf bzw. Wasser aus dem Inneren einer verschlossenen Kammer abgeführt werden.

Günstigerweise ist das Schutzgitter in eine Halterung für das Sterilfilter einsetzbar. Dadurch braucht nur eine Halterung für das Sterilfilter und das Schutzgitter vorgesehen werden.

Günstigerweise weist eine Kammer dem Sterilfilter zugewandt jeweils ein mit Öffnungen versehenes Begrenzungselement auf. Diese Öffnungen erleichtern die Reinigung und Desinfektion der Kammer und durch diese kann auch Wasser bzw. Wasserdampf von der Kammer bei eingelagertem Instrument oder chirurgischem Material abgeführt werden. Konstruktiv besonders einfach ist es, wenn das Begrenzungselement jeweils Teil des Behälterbodens ist.

Günstigerweise ist ein mit Öffnungen versehenes Begrenzungselement für mehrere Kammern einstückig ausgebildet. Dabei sind beispielsweise Spalten zwischen benachbarten Begrenzungselementen vermieden, in denen sich Keime festsetzen können.

Bei einer Variante einer Ausführungsform ist es günstigerweise vorgesehen, daß das mit Öffnungen versehene Begrenzungselement einer Kammer abnehmbar ist. Dadurch wird einerseits der Zusammenbau des erfindungsgemäßen Sterilbehälters erleichtert. Andererseits kann dann das mit den Öffnungen versehene Begrenzungselement bei der Wiederverwertung eines erfindungsgemäßen Sterilbehälters leichter gereinigt werden oder weggeworfen werden und ein neues Begrenzungselement eingesetzt werden. Sind insbesondere die Öffnungen sehr schmal, dann können sich dort Keime festsetzen und statt einer zeitaufwendigen Reinigung und Desinfektion kann es vorteilhafter sein, ein neues Begrenzungselement zu verwenden.

Die nachfolgende Beschreibung bevorzugter Ausführungsformen der Erfindung dient im Zusammenhang mit der Zeichnung der näheren Erläuterung.

Es zeigen:
- Fig. 1:: eine seitliche Schnittdarstellung eines Sterilbehälters;
- Fig. :2: eine Draufsicht auf den Sterilbehälter der Fig. 1;
- Fig. 3:: eine Draufsicht auf ein zweites Ausführungsbeispiel eines Sterilbehälters und
- Fig. 4:: ein weiteres Ausführungsbeispiel eines erfindungsgemäßen Sterilbehälters.

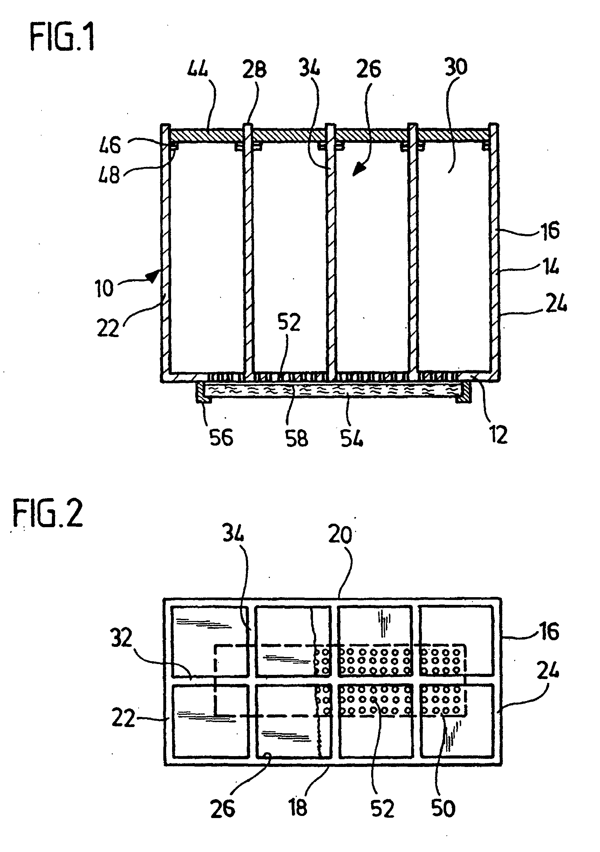

Ein Sterilbehälter, welcher in Fig. 1 als Ganzes mit 10 bezeichnet ist, umfaßt einen Behälterboden 12 und Behälterwände 14. Die Behälterwände selber umfassen wiederum äußere Wände 16, d.h. eine Vorderwand 18, eine Hinterwand 20, eine linke Seitenwand 22 und eine rechte Seitenwand 24 (Fig. 2).

Innerhalb dieser äußeren Wände 18, 20, 22, 24 ist begrenzt durch den Behälterboden 12 ein Aufnahmeraum 26 gebildet. Dieser wiederum ist durch Zwischenwände 28, welche zwischen den äußeren Wänden 16 verlaufen, in eine Mehrzahl von ge. trennten Kammern 30 unterteilt.

Bei dem in den Fig. 1 und 2 gezeigten Ausführungsbeispiel ist der Aufnahmeraum 26 in acht Kammern 30 unterteilt, welche den gleichen Querschnitt und die gleiche Höhe aufweisen. Dazu verläuft mittig zwischen der linken Seitenwand 22 und der rechten Seitenwand 24 eine Längszwischenwand 32 und zwischen der Vorderwand 18 und der Hinterwand 20 verlaufen im gleichen Abstand drei Querzwischenwände 34. Die Zwischenwände 32, 34 sind dabei so ausgebildet, daß die einzelnen Kammern 30 fluiddicht und gasdicht gegeneinander abgeschlossen sind.

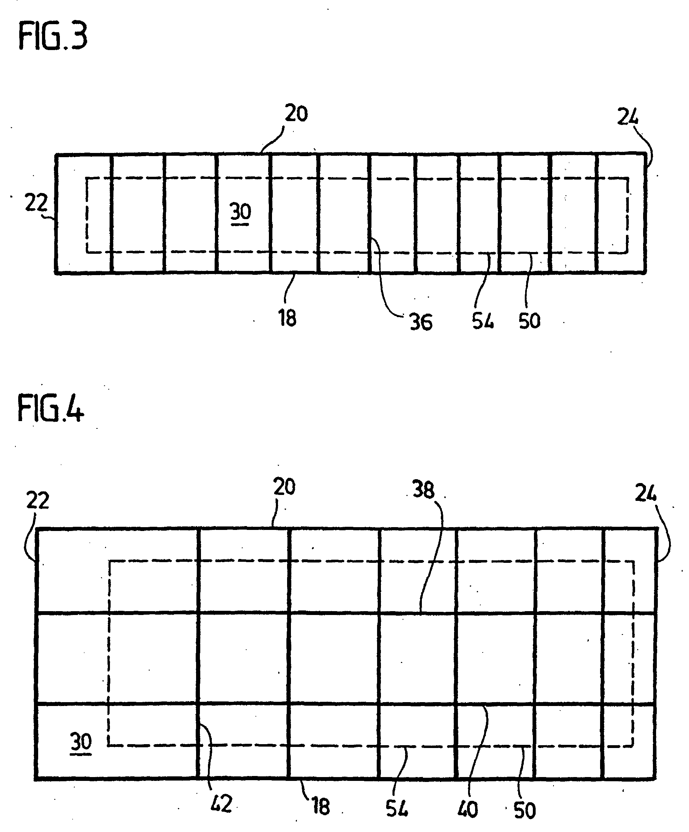

Bei dem in Fig. 3 gezeigten weiteren Ausführungsbeispiel ist eine im Vergleich zu dem ersten Ausführungsbeispiel gemäß Fig. 1, 2 einzeilige Kammeraufteilung gezeigt, bei der keine Längszwischenwand 32 wie in Fig. 2 vorgesehen ist. Durch das Vorsehen einer Mehrzahl von parallelen Querzwischenwänden 36 ist bei diesem Ausführungsbeispiel eine feine Kammerunterteilung erreicht .

Bei dem in Fig. 4 gezeigten Ausführungsbeispiel ist eine erste Längszwischenwand 38 und in einem Abstand zu dieser eine zweite Längszwischenwand 40 parallel zur Vorderwand 18 bzw. zur Hinterwand 20 angeordnet, so daß eine dreizeilige Kammeraufteilung erreicht ist. Querzwischenwände 42 sind in einem Abstand zueinander parallel zur linken Seitenwand 22 bzw. zur rechten Seitenwand 24 angeordnet. Dieser Abstand muß jedoch nicht gleichmäßig sein, so daß einzelne Kammern 30 einen unterschiedlichen Querschnitt aufweisen können. Dadurch läßt sich eine asymmetrische Kammeraufteilung erreichen.

Es versteht sich von selber, daß durch die Wahl der äußeren Abmessungen des erfindungsgemäßen Sterilbehälters, d.h. durch entsprechende Dimensionierung des Behälterbodens 12 und der äußeren Wände 16 sowie entsprechende Anordnung und Dimensionierung der Zwischenwände 28 sich eine Vielzahl von Variationsmöglichkeiten bezüglich der Behälteraufteilung ergeben.

Jede Kammer 30 weist, wie in Fig. 1 gezeigt, ein eigenes Verschlußelement 44 auf, bei dem es sich insbesondere um einen Deckel handelt. Die Kammer 30 ist dadurch bei geschlossenem Verschlußelement 44 von der Außenwelt keimdicht (dicht gegenüber dem Eindringen von Keimen) so abgeschlossen, daß das von einer gereinigten und sterilisierten Kammer aufgenommene ebenfalls sterilisierte Aufnahmegut wie ein chirurgisches Besteck oder anderes chirurgisches Material in der Kammer 30 steril bleibt.

Ein einzelnes Verschlußelement 44 für die zugeordnete Kammer 30 ist beispielsweise an dieser schwenkbar angeordnet (in der Zeichnung nicht gezeigt), so daß die Kammer 30 durch Schwenkung des zugehörigen Verschlußelementes 44 geöffnet werden kann und auf diese Weise auf das in der Kammer 30 steril gelagerte beispielsweise chirurgische Material zugegriffen werden kann. Bei einer alternativen Ausführungsform kann es auch vorgesehen sein, daß ein Verschlußelement 44 mittels einer Schiebeführung geöffnet bzw. geschlossen werden kann. Eine solche Schiebeführung läßt sich insbesondere bei einer einzeiligen Ausführungsform des Sterilbehälters, wie in Fig. 3 gezeigt, einsetzen.

Vorteilhafterweise ist zwischen einem Verschlußelement 44 in dessen geschlossenem Zustand und der Kammer 30 eine Dichtung 46 angeordnet. Beispielsweise weisen dazu die entsprechenden Behälterwände (Seitenwände 22, 24, Querzwischenwände 34, Längszwischenwand 32) entsprechende Vorsprünge 48 auf, die als Auflage für das zugehörige Verschlußelement 44 dienen (bei schwenkbarer Anordnung). Dem Verschlußelement 44 zugewandt sitzt dann auf diesen Vorsprüngen 48 die Dichtung 46, so daß bei geschlossenem Verschlußelement 44 diese eine Abdichtung der Kammer 30 gegenüber dem Außenraum bewirkt. Bei der Dichtung 46 kann es sich um eine unterdruckbeaufschlagte Dichtung handeln, welche insbesondere auf den Vorsprüngen 48 festsitzt (beispielsweise durch eine adhäsive Verbindung) und sich beim Verschließen des Verschlußelementes 44 an diesem festsaugt, um eine erhöhte Dichtigkeit zu erreichen.

Der Behälterboden weist ein Öffnungselement 50 auf, das mit Öffnungen 52 versehen ist, die sich bevorzugterweise parallel zu den Behälterwänden 14 und senkrecht zum Behälterboden 12 durch diesen hindurch erstrecken. Das Öffnungselement 50 ist dabei so dimensioniert, daß jede Kammer 30 des Sterilbehälters 10 in ihrem Boden solche Öffnungen 52 aufweist.

Es kann insbesondere vorgesehen sein, daß das Öffnungselement 50 nicht einstückig mit dem restlichen Sterilbehälter 10 ausgebildet ist, sondern abnehmbar ist.

Zwischen der Außenwelt und den Öffnungen 52 sitzt ein Sterilfilter 54, welches verhindert, daß unsterile Luft über die Öffnungen 52 in die Kammern 30 eindringen kann und so in den Kammern 30 aufbewahrtes Aufnahmegut unsteril macht. Das Sterilfilter 54 ermöglicht aber andererseits einen Durchtritt von Wasserdampf aus den Kammern 30 in den Außenraum. Das Sterilfilter 54 ist insbesondere ein Dauersterilfilter wie beispielsweise ein Filter aus PTFE (Polytetrafluorethylen) oder ein Keramikfilter. Das Sterilfilter 54 ist etwas größer dimensioniert als das Öffnungselement 50, um zu verhindern, daß unsterile Luft in den Sterilbehälter 10 eindringen kann (Fig. 1).

Der erfindungsgemäße Sterilbehälter 10 weist eine Halterung 56 für das Sterilfilter 54 auf. Beispielsweise ist diese so ausgebildet, daß sie einen Schnappverschluß aufweist, mittels welchem sich das Sterilfilter 54 in diese einsetzen läßt und bei Erreichen einer vorbestimmten Position (bei der alle Öffnungen 52 abdeckbar sind) einschnappt, um so einerseits ein schnelles Einsetzen bzw. Wechseln des Sterilfilters 54 zu ermöglichen und andererseits eine sichere Abdeckung der Öffnungen 52 zu erreichen.

Vorteilhafterweise ist die Halterung 56 an dem Sterilbehälter 10 schwenkbar angeordnet, so daß insbesondere während eines Reinigungsvorgangs des Sterilbehälters 10 diese wegschwenkbar ist.

Weiterhin kann es vorgesehen sein, daß zwischen dem Sterilfilter 54 und den Öffnungen 52 in der Halterung 56 ein Schutzgitter 58 sitzt. Dieses Schutzgitter 58, welches beispielsweise aus Metall ist, ist so engmaschig ausgebildet, daß es das Sterilfilter 54 vor einer Penetration durch ein in einer Kammer 30 möglicherweise gelagertes spitzes Instrument schützt. Es kann dabei insbesondere vorgesehen sein, daß die Maschenweite und eine Stegbreite eines maschenbildenden Geflechts so gewählt wird, daß möglichst wenig Rückstände vom Wasch- und Desinfektionsprozeß am Schutzgitter 58 hängenbleiben. Um den Dampfaustausch mit der Außenwelt zu ermöglichen, muß das Schutzgitter 58 dampfdurchlässig sein.

Die Verschlußelemente 44 sind bevorzugterweise zumindest teilweise durchsichtig ausgebildet, so daß ein Bediener auch bei geschlossenem Verschlußelement 44 in einer Kammer 30 aufbewahrtes chirurgisches Besteck oder Material erkennen kann. Es kann auch vorgesehen sein, daß eine oder mehrere Behälterwände 14 durchsichtig ausgebildet sind, um von außen den Blick auf in den Kammern 30 aufbewahrtes Aufnahmegut zu ermöglichen.

Der erfindungsgemäße Sterilbehälter läßt sich wie folgt einsetzen:

Bei geöffneten bzw. abgenommenen Verschlußelementen 44 und bei nicht eingesetztem Sterilfilter 54 bzw. verschwenkter Halterung 56 werden zu sterilisierende Instrumente in die jeweiligen Kammern 30 eingelegt oder befinden sich bereits in den Kammern, in die sie nach Gebrauch zurückgelegt wurden. Der (offene) Sterilbehälter 10 mit der "gefüllten" Kammer 30 wird dann aufbereitet, d.h. es wird ein Reinigungs- und Desinfektionsvorgang durchgeführt. Das Reinigungs- und Desinfektionsfluid kann über die Öffnungen 52 abfließen. Danach werden die Verschlußelemente 44 verschlossen und das Sterilfilter 54 eingesetzt, um daraufhin den gesamten Behälter'zu sterilisieren.

Vor der Verwendung des jeweiligen Instrumentes bzw. chirurgischen Materials öffnet ein Bediener über das zugehörige Verschlußelement 44 nur genau die Kammer 30, die das gewünschte Instrument erhält. Der Bediener kann dies über das durchsichtige Verschlußelement 44 erkennen. Die nicht geöffneten Kammern bleiben steril, d.h. das in ihnen aufgenommene Aufnahmegut wird durch das Öffnen einer anderen Kammer 30 nicht unsteril.

Selbstverständlich können auch andere Reinigungs- und Sterilisationsverfahren als das oben beschriebene automatische Verfahren verwendet werden, um insbesondere wasserempfindliche chirurgische Materialien wie Verbandsmaterialien in den Kammern 30 steril zu lagern.

## Patentansprüche

1. Wiederverwendbarer und wiedersterilisierbarer Sterilbehälter zur Aufnahme und sterilen Aufbewahrung insbesondere von chirurgischem Besteck oder Material mit einem durch einen Behälterboden (12) und Behälterwände (14) gebildeten Aufnahmeraum (26), **dadurch gekennzeichnet, daß** der Aufnahmeraum (26) eine Mehrzahl getrennter Kammern (30) umfaßt, daß jede Kammer (30) einen eigenen Deckel als Verschlußelement (44) aufweist und daß zwischen jeder Kammer (30) und ihrem Verschlußelement (44) eine Dichtung (46) sitzt.

2. Sterilbehälter nach Anspruch 1, **dadurch gekennzeichnet, daß** das Verschlußelement (44) schwenkbar angeordnet ist.

3. Sterilbehälter nach Anspruch 1, **dadurch gekennzeichnet, daß** ein Verschlußelement mittels einer Schiebeführung angeordnet ist.

4. Sterilbehälter nach einem der vorangehenden Ansprüche, **dadurch gekennzeichnet, daß** das Verschlußelement (44) zumindest teilweise durchsichtig ist.

5. Sterilbehälter nach einem der vorangehenden Ansprüche, **dadurch gekennzeichnet, daß** der Sterilbehälter zumindest teilweise aus einem durchsichtigen Material gefertigt ist.

6. Sterilbehälter nach einem der vorangehenden Ansprüche, **dadurch gekennzeichnet, daß** die Dichtung (46) unterdruckbeaufschlagbar ist.

7. Sterilbehälter nach einem der vorangehenden Ansprüche, **dadurch gekennzeichnet, daß** der Sterilbehälter in dem Behälterboden (12) und/oder äußeren Behälterwänden (16) Öffnungen (52) aufweist.

8. Sterilbehälter nach Anspruch 7, **gekennzeichnet durch** ein Sterilfilter (54) zur Abdeckung der Öffnungen (52).

9. Sterilbehälter nach Anspruch 8, **gekennzeichnet durch** ein Sterilfilter (54) für mehrere Kammern (30).

10. Sterilbehälter nach Anspruch 8 oder 9, **dadurch gekennzeichnet, daß** das Sterilfilter (54) unterhalb des Behälterbodens (12) sitzt.

11. Sterilbehälter nach einem der vorangehenden Ansprüche, **dadurch gekennzeichnet, daß** der Sterilbehälter eine Halterung (56) für ein Sterilfilter (54) aufweist.

12. Sterilbehälter nach Anspruch 11, **dadurch gekennzeichnet, daß** die Halterung (56) von dem Sterilbehälter abnehmbar ist.

13. Sterilbehälter nach Anspruch 11 oder 12, **dadurch gekennzeichnet, daß** die Halterung (56) am Sterilbehälter schwenkbar angeordnet ist.

14. Sterilbehälter nach einem der Ansprüche 11 bis 13, **dadurch gekennzeichnet, daß** das Sterilfilter (54) mittels eines Schnappverschlusses in der Halterung (56) gehalten ist.

15. Sterilbehälter nach einem der Ansprüche 8 bis 14, **dadurch gekennzeichnet, daß** zwischen Sterilfilter (54) und Sterilbehälter ein Schutzgitter (58) sitzt.

16. Sterilbehälter nach Anspruch 15, **dadurch gekennzeichnet, daß** das Schutzgitter (58) dampfdurchlässig ist.

17. Sterilbehälter nach Anspruch 15 oder 16, **dadurch gekennzeichnet, daß** das Schutzgitter (58) in eine Halterung (56) für das Sterilfilter (54) einsetzbar ist.

18. Sterilbehälter nach einem der Ansprüche 8 bis 17, **dadurch gekennzeichnet, daß** mindestens eine Kammer (30) dem Sterilfilter (54) zugewandt ein mit Öffnungen (52) versehenes Begrenzungselement (12) aufweist.

19. Sterilbehälter nach Anspruch 18, **dadurch gekennzeichnet, daß** das Begrenzungselement jeweils Teil (50) des Behälterbodens (12) ist.

20. Sterilbehälter nach Anspruch 19, **dadurch gekennzeichnet, daß** ein mit Öffnungen (52) versehenes Begrenzungselement für mehrere Kammern (30) einstückig ausgebildet ist.

21. Sterilbehälter nach einem der Ansprüche 18 bis 20, **dadurch gekennzeichnet, daß** das mit Öffnungen (52) versehene Begrenzungselement (12) der mindestens einen Kammer (30) abnehmbar ist.

## Claims

1. A reusable and resterilisable sterile container for holding and for sterile storage of, in particular, surgical instruments or materials, comprising a holding space (26) formed by a container base (12) and container walls (14), **characterised in that** the holding space (26) comprises a plurality of separate chambers (30), **in that** each chamber (30) has its own cover forming a closure (44) and **in that** a seal (46) is arranged between each chamber (30) and its closure (44).

2. A sterile container according to claim 1, **characterised in that** the closure (44) is pivotably arranged.

3. A sterile container according to claim 1, **characterised in that** a closure is slidably arranged.

4. A sterile container according to any one of the preceding claims, **characterised in that** the closure (44) is at least partly transparent.

5. A sterile container according to any one of the preceding claims, **characterised in that** the sterile container is manufactured at least in part from a transparent material.

6. A sterile container according to any one of the preceding claims, **characterised in that** the seal (46) is compressible.

7. A sterile container according to any one of the preceding claims, **characterised in that** the sterile container has openings (52) in the container base (12) and/or outer container walls (16).

8. A sterile container according to claim 7, **characterised by** a sterile filter (54) for covering the openings (52).

9. A sterile container according to claim 8, **characterised by** a sterile filter (54) for a plurality of chambers (30).

10. A sterile container according to claim 8 or 9, **characterised in that** the sterile filter (54) is arranged underneath the container base (12).

11. A sterile container according to any one of the preceding claims, **characterised in that** the sterile container has a mounting (56) for a sterile filter (54).

12. A sterile container according to claim 11, **characterised in that** the mounting (56) is detachable from the sterile container.

13. A sterile container according to claim 11 or 12, **characterised in that** the mounting (56) is pivotably arranged on the sterile container.

14. A sterile container according to any one of claims 11 to 13, **characterised in that** the sterile filter (54) is held in the mounting (56) by means of a snap fastening.

15. A sterile container according to any one of claims 8 to 14, **characterised in that** a protective screen (58) is arranged between the sterile filter (54) and the sterile container.

16. A sterile container according to claim 15, **characterised in that** the protective screen (58) is vapour-permeable.

17. A sterile container according to claim 15 or 16, **characterised in that** the protective screen (58) is insertable into a mounting (56) for the sterile filter (54).

18. A sterile container according to any one of claims 8 to 17, **characterised in that** at least one chamber (30) has a peripheral component (12) facing the sterile filter (54) and provided with openings (52).

19. A sterile container according to claim 18, **characterised in that** each peripheral component is part (50) of the container base (12).

20. A sterile container according to claim 19, **characterised in that** a peripheral component provided with openings (52) is formed in one piece for a plurality of chambers (30).

21. A sterile container according to any one of claims 18 to 20, **characterised in that** the peripheral component (12) provided with openings (52) is removable from the at least one chamber (30).

## Revendications

1. Récipient stérile réutilisable et restérilisable destiné à recevoir et à conserver de manière stérile en particulier des ustensiles chirurgicaux ou du matériel chirurgical comportant un espace de réception (26) formé par un fond de récipient (12) et des parois de récipient (14), **caractérisé en ce que** l'espace de réception (26) comprend une multiplicité de chambres (30) séparées, **en ce que** chaque chambre (30) comporte un couvercle spécifique à titre d'élément de fermeture (44) et **en ce qu'**un joint d'étanchéité (46) est situé entre chaque chambre (30) et son élément de fermeture (44).

2. Récipient stérile selon la revendication 1, **caractérisé en ce que** l'élément de fermeture (44) est disposé de manière pivotable.

3. Récipient stérile selon la revendication 1, **caractérisé en ce qu'**un élément de fermeture est disposé au moyen d'un guide coulissant.

4. Récipient stérile selon l'une des revendications précédentes, **caractérisé en ce que** l'élément de fermeture (44) est transparent au moins en partie.

5. Récipient stérile selon l'une des revendications précédentes, **caractérisé en ce que** le récipient stérile est constitué au moins en partie par un matériau transparent.

6. Récipient stérile selon l'une des revendications précédentes, **caractérisé en ce que** le joint d'étanchéité (46) peut être soumis à une pression négative.

7. Récipient stérile selon l'une des revendications précédentes, **caractérisé en ce que** le récipient stérile comporte des ouvertures (52) dans le fond de récipient (12) et/ou dans les parois de récipient externes (16).

8. Récipient stérile selon la revendication 7, **caractérisé par** un filtre stérile (54) destiné à recouvrir les ouvertures (52).

9. Récipient stérile selon la revendication 8, **caractérisé par** un filtre stérile (54) pour plusieurs chambres (30).

10. Récipient stérile selon la revendication 8 ou 9, **caractérisé en ce que** le filtre stérile (54) est situé en-dessous du fond de récipient (12).

11. Récipient stérile selon l'une des revendications précédentes, **caractérisé en ce que** le récipient stérile comporte un support (56) pour un filtre stérile (54).

12. Récipient stérile selon la revendication 11, **caractérisé en ce que** le support (56) peut être retiré du récipient stérile.

13. Récipient stérile selon la revendication 11 ou 12, **caractérisé en ce que** le support (56) est disposé de manière pivotable sur le récipient stérile.

14. Récipient stérile selon l'une des revendications 11 à 13, **caractérisé en ce que** le filtre stérile (54) est maintenu dans le support (56) au moyen d'une fermeture à enclenchement.

15. Récipient stérile selon l'une des revendications 8 à 14, **caractérisé en ce qu'**une grille protectrice (58) est située entre le filtre stérile (54) et le récipient stérile.

16. Récipient stérile selon la revendication 15, **caractérisé en ce que** la grille protectrice (58) est perméable à la vapeur.

17. Récipient stérile selon la revendication 15 ou 16, **caractérisé en ce que** la grille protectrice (58) peut être insérée dans un support (56) pour le filtre stérile (54).

18. Récipient stérile selon l'une des revendications 8 à 17, **caractérisé en ce qu'**au moins une chambre (30) comporte un élément de limitation (12) muni d'ouvertures (52) tourné vers le filtre stérile (54).

19. Récipient stérile selon la revendication 18, **caractérisé en ce que** l'élément de limitation fait chaque fois partie (50) du fond de récipient (12).

20. Récipient stérile selon la revendication 19, **caractérisé en ce qu'**un élément de limitation muni d'ouvertures (52) est formé d'une pièce pour plusieurs chambres (30).

21. Récipient stérile selon l'une des revendications 18 à 20, **caractérisé en ce que** l'élément de limitation (12) muni d'ouvertures (52) d'au moins une chambre (30) est amovible.
